# EUROPEAN PATENT SPECIFICATION

(11) **EP 0 712 406 B1**
(45) Date of publication and mention of the grant of the patent: **28.10.1998**
(21) Application number: 94926128.3
(22) Date of filing: 28.07.1994
(51) Int. Cl.: C07D 498/04, A61K 31/535

(54) **CONDENSED INDOLE DERIVATIVES AS 5-HT 4-RECEPTOR ANTAGONISTS**
KONDENSIERTE INDOL-DERIVATE ALS 5-HT4-REZEPTOR-ANTAGONISTEN
DERIVES CONDENSES D'INDOLES EN TANT QU'ANTAGONISTES DU RECEPTEUR DE 5-HT 4

(30) Priority: 05.08.1993 GB 9316195
(43) Date of publication of application: 22.05.1996
(73) Proprietor: SMITHKLINE BEECHAM PLC, Brentford, Middlesex TW8 9EP (GB)
(72) Inventor: GASTER, Laramie, Mary, The Pinnacles Harlow Essex CM19 5AD (GB); WYMAN, Paul, Adrian, The Pinnacles Harlow Essex CM 19 5AD (GB)
(74) Representative: Tocher, Pauline
(86) International application number: EP9402514
(87) International publication number: WO9504737

(56) References cited:
- EP-A- 0 150 505
- WO-A-93/18036

## Description

This invention relates to novel compounds having pharmacological activity, to a process for their preparation and to their use in the manufacture of medicaments.

EP-A-429984 (Nisshin Flour Milling Co., Ltd.) describes indole derivatives having 5-HT₃ receptor antagonist activity.

European Journal of Pharmacology 146 (1988), 187-188, and Naunyn-Schmiedeberg's Arch. Pharmacol. (1989) 340:403-410, describe a non classical 5-hydroxytryptamine receptor, now designated the 5-HT₄ receptor, and that ICS 205-930, which is also a 5-HT₃ receptor antagonist, acts as an antagonist at this receptor.

WO 91/16045 (SmithKline and French Laboratories Limited) describes the use of cardiac 5-HT₄ receptor antagonists in the treatment of atrial arrhythmias and stroke.

EP-A-501322 (Glaxo Group Limited) describes indole derivatives having 5-HT₄ antagonist activity.

WO 93/18036 (SmithKline Beecham plc) describes compounds having 5-HT₄ receptor antagonist activity of formula (I), or a pharmaceutically acceptable salt thereof: 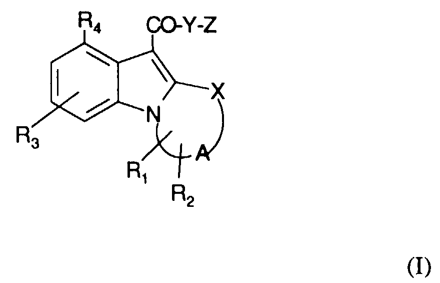 wherein
X is O, S, SO, SO₂, CH₂, CH or NR wherein R is hydrogen or C₁₋₆ alkyl;
A is a saturated or unsaturated polymethylene chain of 2 - 4 carbon atoms;
R₁ and R₂ are hydrogen or C₁₋₆ alkyl;
R₃ is hydrogen, halo, C₁₋₆ alkyl, amino, nitro or C₁₋₆ alkyl;
R₄ is hydrogen, halo, C₁₋₆ alkyl or C₁₋₆ alkoxy;
Y is O or NH;
Z is of sub-formula (a), (b) or (c): 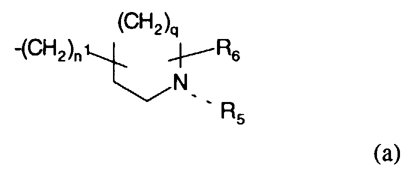 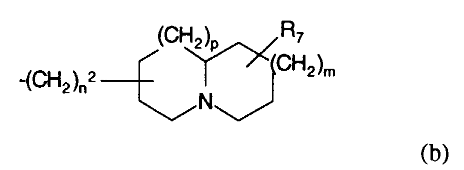 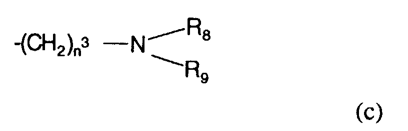
wherein
n¹ is 1, 2, 3 or 4; n² is 0, 1, 2, 3 or 4; n³ is 2, 3, 4 or 5;
q is 0, 1, 2 or 3; p is 0, 1 or 2; m is 0, 1 or 2;
R₅ is hydrogen, C₁₋₁₂ alkyl, aralkyl or R₅ is (CH₂)_{z}-R₁₀ wherein z is 2 or 3 and R₁₀ is selected from cyano, hydroxyl, C₁₋₆ alkoxy, phenoxy, C(O)C₁₋₆ alkyl, COC₆H₅, -CONR₁₁R₁₂, NR₁₁COR₁₂, SO₂NR₁₁R₁₂ or NR₁₁SO₂R₁₂ wherein R₁₁ and R₁₂ are hydrogen or C₁₋₆ alkyl; and
R₆, R₇ and R₈ are independently hydrogen or C₁₋₆ alkyl; and
R₉ is hydrogen or C₁₋₁₀ alkyl;
or a compound of formula (I) wherein the CO-Y linkage is replaced by a heterocyclic bioisostere;
having 5-HT₄ receptor antagonist activity.

In one aspect of the present invention there is provided a compound as claimed in claim 1.

Examples of alkyl or alkyl containing groups include C₁, C₂, C₃, C₄, C₅ or C₆ branched, straight chained or cyclic alkyl, as appropriate. C₁₋₄ alkyl groups include methyl, ethyl, *n-* and iso-propyl, *n-, iso-, sec-* and *tert*-butyl. Cyclic alkyl includes cyclopropyl, cyclobutyl, cyclopentyl, cyclohexyl, cycloheptyl and cyclooctyl.

Halo includes fluoro, chloro, bromo and iodo.

X is often O.

Values for A include -CH₂-(CH₂)ᵣ-CH₂- wherein r is 0, 1 or 2; -CH₂-CH=CH-; -C(CH₃)=CH- or when X is CH or NR, A may be -(CH₂)₂-CH= or -CH=CH-CH=.

R₁ and R₂ are often hydrogen or R₁ and R₂ are gem-dimethyl.

r is often 1.

R₃ is preferably hydrogen.

R₄ is preferably hydrogen or halo, such as fluoro.

n¹ is preferably 2, 3 or 4 when the azacycle is attached at the nitrogen atom, and n¹ is preferably 1 when the azacycle is attached at a carbon atom, such as the 4-position when q is 2.

Preferably, R₁, R₂, R₃ and R₄ are all H, r is 1, X is O and Y is NH.

The pharmaceutically acceptable salts of the compounds of the formula (IA) include acid addition salts with conventional acids such as hydrochloric, hydrobromic, boric, phosphoric, sulphuric acids and pharmaceutically acceptable organic acids such as acetic, tartaric, maleic, citric, succinic, benzoic, ascorbic, methanesulphonic, α-keto glutaric, α-glycerophosphoric, and glucose-1-phosphoric acids.

Examples of pharmaceutically acceptable salts include quaternary derivatives of the compounds of formula (IA) such as the compounds quaternised by compounds Rₓ-T wherein Rₓ is C₁₋₆ alkyl, phenyl-C₁₋₆ alkyl or C₅₋₇ cycloalkyl, and T is a radical corresponding to an anion of an acid. Suitable examples of Rₓ include methyl, ethyl and *n-* and *iso*-propyl; and benzyl and phenethyl. Suitable examples of T include halide such as chloride, bromide and iodide.

Examples of pharmaceutically acceptable salts also include internal salts such as N-oxides.

The compounds of the formula (IA), their pharmaceutically acceptable salts, (including quaternary derivatives and N-oxides) may also form pharmaceutically acceptable solvates, such as hydrates, which are included wherever a compound of formula (I) or a salt thereof is herein referred to.

The compounds of formula (IA) may be prepared by conventional coupling of the indole moiety with Z. Suitable methods are as referred to in WO 93/18036.

The compounds of the present invention are 5-HT₄ receptor antagonists and it is thus believed may generally be used in the treatment or prophylaxis of gastrointestinal disorders, cardiovascular disorders and CNS disorders.

They are of potential interest in the treatment of irritable bowel syndrome (IBS), in particular the diarrhoea aspects of IBS, i.e., these compounds block the ability of 5-HT to stimulate gut motility via activation of enteric neurones. In animal models of IBS, this can be conveniently measured as a reduction of the rate of defaecation. They are also of potential use in the treatment of urinary incontinence which is often associated with IBS.

They may also be of potential use in other gastrointestinal disorders, such as those associated with upper gut motility, and as antiemetics. In particular, they are of potential use in the treatment of the nausea and gastric symptoms of gastro-oesophageal reflux disease and dyspepsia. Antiemetic activity is determined in known animal models of cytotoxic-agent/radiation induced emesis.

Specific cardiac 5-HT₄ receptor antagonists which prevent atrial fibrillation and other atrial arrhythmias associated with 5-HT, would also be expected to reduce occurrence of stroke (see A.J. Kaumann 1990, Naumyn-Schmiedeberg's Arch. Pharmacol. 342, 619-622, for appropriate animal test method).

Anxiolytic activity is likely to be effected via the hippocampus (Dumuis *et al* 1988, Mol Pharmacol., 34, 880-887). Activity can be demonstrated in standard animal models, the social interaction test and the X-maze test.

Migraine sufferers often undergo situations of anxiety and emotional stress that precede the appearance of headache (Sachs, 1985, Migraine, Pan Books, London). It has also been observed that during and within 48 hours of a migraine attack, cyclic AMP levels are considerably increased in the cerebrospinal fluid (Welch *et al.,* 1976, Headache 16, 160-167). It is believed that a migraine, including the prodomal phase and the associated increased levels of cyclic AMP are related to stimulation of 5-HT₄ receptors, and hence that administration of a 5-HT₄ antagonist is of potential benefit in relieving a migraine attack.

Other CNS disorders of interest include schizophrenia, Parkinson's disease and Huntingdon's chorea.

The invention also provides a pharmaceutical composition comprising a compound of formula (IA), or a pharmaceutically acceptable salt thereof, and a pharmaceutically acceptable carrier.

Such compositions are prepared by admixture and are usually adapted for enteral such as oral, nasal or rectal, or parenteral administration, and as such may be in the form of tablets, capsules, oral liquid preparations, powders, granules, lozenges, reconstitutable powders, nasal sprays, suppositories, injectable and infusable solutions or suspensions. Orally administrable compositions are preferred, since they are more convenient for general use.

Tablets and capsules for oral administration are usually presented in a unit dose, and contain conventional excipients such as binding agents, fillers, diluents, tabletting agents, lubricants, disintegrants, colourants, flavourings, and wetting agents. The tablets may be coated according to well known methods in the art, for example with an enteric coating.

Suitable fillers for use include cellulose, mannitol, lactose and other similar agents. Suitable disintegrants include starch, polyvinylpolypyrrolidone and starch derivatives such as sodium starch glycollate. Suitable lubricants include, for example, magnesium stearate.

Suitable pharmaceutically acceptable wetting agents include sodium lauryl sulphate. Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethylcellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl p-hydroxybenzoate or sorbic acid, and if desired conventional flavouring or colouring agents.

Oral liquid preparations are usually in the form of aqueous or oily suspensions, solutions, emulsions, syrups, or elixirs or are presented as a dry product for reconstitution with water or other suitable vehicle before use. Such liquid preparations may contain conventional additives such as suspending agents, emulsifying agents, non-aqueous vehicles (which may include edible oils), preservatives, and flavouring or colouring agents.

The oral compositions may be prepared by conventional methods of blending, filling or tabletting. Repeated blending operations may be used to distribute the active agent throughout those compositions employing large quantities of fillers. Such operations are, of course, conventional in the art.

For parenteral administration, fluid unit dose forms are prepared containing a compound of the present invention and a sterile vehicle. The compound, depending on the vehicle and the concentration, can be either suspended or dissolved. Parenteral solutions are normally prepared by dissolving the compound in a vehicle and filter sterilising before filling into a suitable vial or ampoule and sealing. Advantageously, adjuvants such as a local anaesthetic, preservatives and buffering agents are also dissolved in the vehicle. To enhance the stability, the composition can be frozen after filling into the vial and the water removed under vacuum.

Parenteral suspensions are prepared in substantially the same manner except that the compound is suspended in the vehicle instead of being dissolved and sterilised by exposure of ethylene oxide before suspending in the sterile vehicle. Advantageously, a surfactant or wetting agent is included in the composition to facilitate uniform distribution of the compound of the invention.

An amount effective to treat the disorders hereinbefore described depends on the relative efficacies of the compounds of the invention, the nature and severity of the disorder being treated and the weight of the mammal. However, a unit dose for a 70kg adult will normally contain 0.05 to 1000mg for example 0.5 to 500mg, of the compound of the invention. Unit doses may be administered once or more than once a day, for example, 2, 3 or 4 times a day, more usually 1 to 3 times a day, that is in the range of approximately 0.0001 to 50mg/kg/day, more usually 0.0002 to 25 mg/kg/day.

No adverse toxicological effects are indicated within the aforementioned dosage ranges.

In another aspect of the present invention there is provided the use of a compound according to the invention in the manufacture of a medicament for use as a 5-HT₄ receptor antagonist.

Yet another aspect of the present invention comprehends a process for preparing compounds according to the invention which process comprises reacting an appropriate acid derivative with an appropriate alcohol or amine.

The invention also provides a compound of formula (IA) or a pharmaceutically acceptable salt thereof for use as an active therapeutic substance, in particular for use in the treatment of irritable bowel syndrome, gastro-oesophageal reflux disease, dyspepsia, atrial arrhythmias and stroke, anxiety and/or migraine.

The following Example illustrates the preparation of compounds of formula (IA), the following Descriptions relate to the preparation of intermediates.

### Example

### N-[(1-(3-Phenoxypropyl)-4-piperidinyl)methyl]3,4-dihydro-2H-[1,3]oxazino[3,2-a]indole-10-carboxamide [X = O, A = (CH₂)₃, (R₁, R₂, R₃, R₄ = H; Y = NH, Z = 4-piperidinylmethyl, R₅ = 3-phenoxypropyl]

A stirred solution of N-(4-piperidinylmethyl) 3,4-dihydro-2H-[1,3]oxazino[3,2-a]indole-10-carboxamide (D2) (250mg, 0.80 mmole) and triethylamine (0.25 ml, 1.8 mmole) in a mixture of acetonitrile (15 ml) and N,N-dimethylformamide (10ml) was treated with 3-phenoxypropyl bromide (0.13 ml, 0.88 mmole) and the solution heated under reflux for 48h. The mixture was allowed to cool, then concentrated *in vacuo* and the residue dissolved in ethyl acetate (25 ml) and washed with water (20 ml). The organic solution was dried (MgSO₄), concentrated *in vacuo* and the residue purified by flash chromatography on silica gel eluting with 0 - 20% methanol/ethyl acetate to afford the title compound as a pale pink solid after trituation with ether (44mg) mp 120-126°C.
¹H NMR (CDCl₃)
δ: 8.30(d,1H), 7.07-7.37(m,5H), 6.96(t,1H), 6.88(d,2H), 6.64(t,1H), 4.57(t,2H), 4.13(t,2H), 4.30(t,2H), 3.22-3.43(m,4H), 2.78-3.00(m,2H), 2.13-2.57(m,6H), 1.59-2.03(m,5H).

### Description 1

### (1-Benzyl-4-piperidinyl)methylamine(D1)

A stirred solution of isonipecotamide (30.1g, 0.23 mole) and benzyl bromide (27.9 ml, 0.23 mole) in ethanol (250 ml) was treated with anhydrous potassium carbonate (64.9g, 0.47 mole) and heated under reflux for 3h. The mixture was allowed to cool, then filtered and the filtrate concentrated under vacuum. The residual oil was dissolved in chloroform (200 ml) and washed with water (1 x 150 ml), then dried (Na₂SO₄) and concentrated under vacuum to leave a yellow solid (41.0g). This solid was mixed thoroughly with phosphorus pentoxide (38.3g, 0.27 mole) and the mixture heated at 180°C under nitrogen for 2.5h with gentle stirring. The reaction mixture was allowed to cool, then treated with water (300 ml). When the solid mass had dissolved, the solution was basified by addition of solid K₂CO₃ and extracted with ethyl acetate (2x250 ml). The combined extracts were dried (Na₂SO₄) and concentrated *in vacuo* to leave a brown oil (35.3g). This was dissolved in dry ether (250 ml) and added dropwise over 30 minutes to a stirred suspension of lithium aluminium hydride (10.1g, 0.26 mole) in ether (150ml) at 0°C under nitrogen. When addition was complete, the mixture was allowed to warm up to room temperature and was stirred for 1.5h. It was re-cooled to 0°C and treated cautiously with water (10ml), 10% NaOH solution (15 ml) and water again (25ml). The mixture was filtered through kieselguhr and the filtrate concentrated *in vacuo* to leave a brown oil, which was distilled under vacuum to afford the title compound as a colourless oil after distillation (27.8g, 67%) bp 106°C at 0.25 mmHg.
¹H NMR (CDCl₃)
δ: 7.20-7.37(m,5H), 3.48(s,2H), 2.85-2.95(m,2H), 2.55(d,2H), 1.87-2.00(m,2H), 1.60-1.75(m,2H), 1.10-1.40(m,5H).

### Description 2

### a) N-[(1-Benzyl-4-piperidinyl)methyl]indole-3-carboxamide

To a stirred solution of indole-3-carboxylic acid (15g, 0.093mole) in dichloromethane (250 ml) under nitrogen was added oxalyl chloride (8.7 ml, 0.10 mole) and dry dimethylformamide (6 drops). After 2 hours, the solvent was evaporated under reduced pressure. The residual acid chloride (0.093 mole) was dissolved in dichloromethane (100 ml) and added dropwise to a stirred solution of N-(1-benzyl-4-piperidinyl)methylamine (D1, 16.4g, 0.093 mole) and triethylamine (15.5 ml, 0.11 mole) in dichloromethane (150 ml) at 5°C. After stirring at ambient temperature overnight, the reaction mixture was washed with 10% Na₂CO₃ and the organic phase was dried (Na₂SO₄). The solvent was evaporated under reduced pressure and the residual solid recrystallised from ethyl acetate to afford the title compound as a white solid (17.5g, 60%).
¹H NMR (CDCl₃)
δ: 9.90(s,1H), 7.85-7.95(m,1H), 7.64(d,lH), 7.15-7.43(m,8H), 6.17(t,1H), 3.48(s,2H), 3.37(t,2H), 2.83-2.98(m,2H), 1.87-2.08(m,2H), 1.54-1.82(m,3H), 1.23-1.50(m,2H).

### b) N-[(1-Benzyl-4-piperidinyl)methyl]3,4-dihydro-2H-[1,3]oxazino[3,2-a]indole-10-carboxamide

A stirred suspension of N-[(1-benzyl-4-piperidinyl)methyl] indole-3-carboxamide (17.5g, 0.050 mole) in chloroform (250 ml) was treated with 3-bromo-1-propanol (10.1 ml, 0.11 mole) and N-chlorosuccinimide (8.7g, 0.065 mole) at room temperature and a clear solution was obtained in 15 minutes. After lh the reaction mixture darkened in colour from pale yellow to orange and temperature rose to 38°C. After a further 1 h the reaction mixture was treated with 10% NaHCO₃ solution and the chloroform layer separated, dried (Na₂SO₄) and concentrated *in vacuo* to leave a yellow oil, which was chromatographed on silica gel eluting with 3% methanol/chloroform. The 2-(3-bromopropoxy)indole intermediate was dissolved in acetone (400 ml), treated with anhydrous potassium carbonate (11g, 0.08 mole) and stirred at room temperature for 20h. The reaction mixture was concentrated *in vacuo* and the residue treated with water (200 ml) and extracted with chloroform (2 x 250 ml). The combined extracts were dried (Na₂SO₄), concentrated in *vacuo* and the residue chromatographed on silica gel eluting with 5% methanol/chloroform to afford the title compound as a pale yellow oil (3.1g, 15%). This was converted to its oxalate salt and crystallised from acetone as a white solid mp 169-170°C.
Free base:- ¹H NMR (CDCl₃)
δ: 8.32(d,1H), 7.05-7.38(m,8H), 6.53(t,1H), 4.50(t,2H), 4.08(t,2H), 3.48(s,2H), 3.31(t,2H), 2.83-2.97(m,2H), 2.27-2.41(m,2H), 1.54-2.06(m,5H), 1.25-1.45(m,2H).

### c) N-(4-Piperidinylmethyl) 3,4-dihydro-2H-[1,3]oxazino[3,2-a]indole-10-carboxamide

A stirred suspension of N-[(1-benzyl-4-piperidinyl)methyl] 3,4-dihydro-2H-[1,3]oxazino[3,2-a]indole-10-carboxamide oxalate salt (2.25g, 0.0046 mole) in ethanol (100 ml) and glacial acetic acid (4 ml) was hydrogenated over 10% Pd-C (0.8g) at atmospheric pressure and 45°C for 18h. The mixture was filtered and the filtrate concentrated *in vacuo.* The majority of the product was in the solid which had been filtered off. This material was shaken with concentrated potassium carbonate solution (50 ml) and chloroform (50 ml) together with the residue from the filtrate. The mixture was filtered, the chloroform layer separated and dried (Na₂SO₄), then concentrated *in vacuo* to afford the title compound (D2) as a white solid (1.52g, 100%). This was recrystallised from chloroform/60-80 petrol mp 139-141°C.
¹H NMR (CDCl₃)
δ: 8.32(d,1H), 7.03-7.30(m,3H), 6.53(t,1H), 4.48(t,2H), 4.05(t,2H), 3.30(t,2H), 3.02-3.15(m,2H), 2.52-2.70(m,2H), 2.27-2.40(m,2H), 1.65-1.90(m,4H), 1.10-1.30(m,2H).

### 5-HT₄ RECEPTOR ANTAGONIST ACTIVITY

### 1) Guinea pig colon

Male guinea-pigs, weighing 250-400g are used. Longitudinal muscle-myenteric plexus preparations, approximately 3cm long, are obtained from the distal colon region. These are suspended under a 0.5g load in isolated tissue baths containing Krebs solution bubbled with 5% CO₂ in O₂ and maintained at 37°C. In all experiments, the Krebs solution also contains methiothepin 10⁻⁷M and granisetron 10⁻⁶M to block effects at 5-HT₁, 5-HT₂ and 5-HT₃ receptors.

After construction of a simple concentration-response curve with 5-HT, using 30s contact times and a 15min dosing cycle, a concentration of 5-HT is selected so as to obtain a contraction of the muscle approximately 40-70% maximum(10⁻⁹M approx). The tissue is then alternately dosed every 15min with this concentration of 5-HT and then with an approximately equi-effective concentration of the nicotine receptor stimulant, dimethylphenylpiperazinium (DMPP). After obtaining consistent responses to both 5-HT and DMPP, increasing concentrations of a putative 5-HT₄ receptor antagonist are then added to the bathing solution. The effects of this compound are then determined as a percentage reduction of the contractions evoked by 5-HT or by DMPP. From this data, pIC₅₀ values are determined, being defined as the -log concentration of antagonist which reduces the contraction by 50%. A compound which reduces the response to 5-HT but not to DMPP is believed to act as a 5-HT₄ receptor antagonist.

The compound had a pIC₅₀ of 9.65 ± 0.20 (n = 4).

### 2) Rat oesophagus

Rat oesophageal tunica muscularis mucosae is set up according to Baxter *et. al.* Naunyn-Schmiedeberg's Arch. Pharmacol., 343, 439-446 (1991). The inner smooth muscle tube of the muscularis mucosae is isolated and mounted for isometric tension recording in oxygenated (95% O₂/5% CO₂) Tyrodes solution at 37°C. All experiments are performed in pargyline pre-treated preparations (100µM for 15 min followed by washout) and in the presence of cocaine (30µM). Relaxant responses to 5-HT are obtained after pre-contracting the oesophagus tissue with carbachol (3µM).

## Claims

1. A compound of formula (IA) or a pharmaceutically acceptable salt thereof: 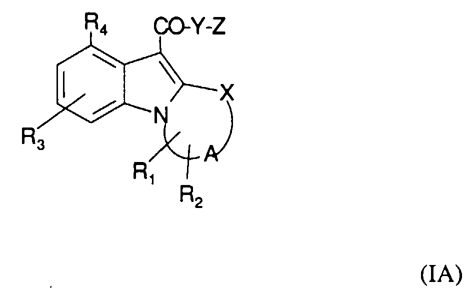 wherein
X is O, S, SO, SO₂, CH₂, CH or NR wherein R is hydrogen or C₁₋₆ alkyl;
A is a saturated or unsaturated polymethylene chain of 2 - 4 carbon atoms;
R₁ and R₂ are hydrogen or C₁₋₆ alkyl;
R₃ is hydrogen, halo, C₁₋₆ alkyl, amino, nitro or C₁₋₆ alkyl;
R₄ is hydrogen, halo, C₁₋₆ alkyl or C₁₋₆ alkoxy;
Y is O or NH;
Z is of sub-formula (a): 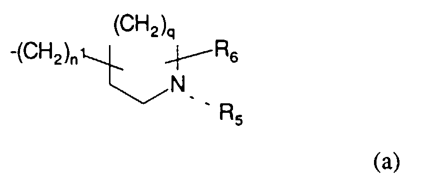 wherein
n¹ is 1, 2, 3 or 4;
q is 0, 1, 2 or 3;
R₅ is 3-phenoxypropyl;
R₆ is hydrogen or C₁₋₆ alkyl.

2. A compound according to claim 1 wherein X is O.

3. A compound according to claim 1 or 2 wherein A is -(CH₂)₃-.

4. A compound according to claim 1, 2 or 3 wherein R₁ and R₂ are independently hydrogen or methyl.

5. A compound according to claim 1, 2, 3 or 4 wherein R₃ is hydrogen and R₄ is hydrogen or halo.

6. A compound according to any one of claims 1 to 5 wherein Y is NH.

7. A compound according to any one of claims 1 to 6 wherein Z is of sub-formula (a) and (CH₂)ₙ1 is attached at a carbon atom of the azacycle.

8. A compound according to claim 7 wherein Z is N-substituted 4-piperidylmethyl.

9. N-[(1-(3-Phenoxypropyl)-4-piperidinyl)methyl] 3,4-dihydro-2H-[1,3]oxazino[3,2-a]indole-10-carboxamide or a pharmaceutically acceptable salt thereof.

10. A process for preparing compounds according to claim 1, which comprises reacting an appropriate acid derivative with an appropriate alcohol or amine.

11. A pharmaceutical composition comprising a compound according to any one of claims 1 to 9, and a pharmaceutically acceptable carrier.

12. A compound according to claim 1 for use as an active therapeutic substance.

13. The use of a compound according to claim 1 in the manufacture of a medicament for use as a 5-HT₄ receptor antagonist.

14. The use according to claim 13 for use as a 5-HT₄ antagonist in the treatment or prophylaxis of gastrointestinal disorders, cardiovascular disorders and CNS disorders.

15. The use according to claim 13 for use in the treatment of irritable bowel syndrome, urinary incontinence or atrial arrhythmias and stroke.

## Patentansprüche

1. Eine Verbindung der Formel (IA) oder ein pharmazeutisch verträgliches Salz davon 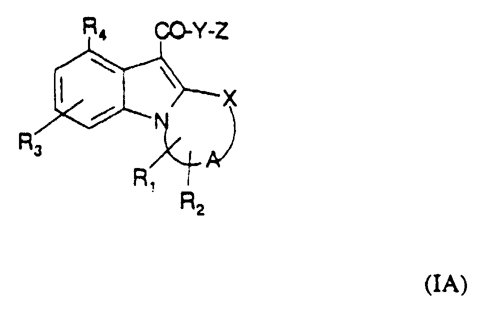 wobei
X die Gruppe O, S, SO, SO₂, CH₂,CH oder NR ist, wobei R ein Wasserstoffatom oder ein C₁₋₆-Alkylrest ist,
A eine gesättigte oder ungesättigte Polymethylenkette mit 2-4 Kohlenstoffatomen ist,
R₁ und R₂ ein Wasserstoffatom oder ein C₁₋₆-Alkylrest sind,
R₃ ein Wasserstoff-, Halogenatom-, C₁₋₆-Alkyl-, Amino-, Nitro- oder C₁₋₆-Alkoxyest ist,
R₄ ein Wasserstoff-, Halogenatom, C₁₋₆-Alkyl- oder C₁₋₆-Alkoxyrest ist,
Y ein O-Atom oder eine NH-Gruppe ist,
Z dieUnterformel (a) bedeutet 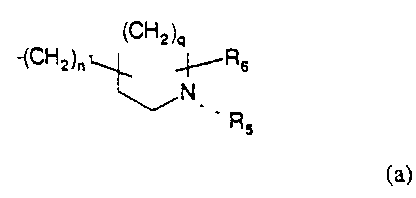 wobei
n¹ der Wert 1, 2, 3 oder 4 ist,
q der Wert 0, 1, 2 oder 3 ist
R₅ eine 3-Phenoxypropylgruppe ist,
R₆ ein Wasserstoffatom oder ein C₁₋₆-Alkylrest ist.

2. Eine Verbindung gemäß Anspruch 1, wobei X ein O-Atom ist

3. Eine Verbindung gemäß Anspruch 1 oder 2, wobei A -(CH₂)₃- ist .

4. Eine Verbindung, gemäß dem Anspruch 1, 2 oder 3, wobei R₁ und R₂ unabhängige Wasserstoffatome oder Methylgruppen sind.

5. Einer Verbindung, gemäß Anspruch 1, 2, 3 oder 4, wobei R₃ ein Wasserstoffatom und R₄ ein Wasserstoff- oder ein Halogenatom ist.

6. Eine Verbindung, gemäß einem der Ansprüche 1 bis 5, wobei Y eine NH-Gruppe ist.

7. Eine Verbindung gemäß einem der Ansprüche 1 bis 5, wobei Z die Unterformel (a) bedeutet und (CH₂)ₙ1 an einem Kohlenstoffatom des azacyclischen Rests gebunden ist.

8. Eine Verbindung, gemäß Anspruch 7, wobei Z ein N-substituierter 4-Piperidylmethylrest ist.

9. N[(1-(3-Phenoxypropyl)-4-piperidinyl)methyl]-3,4-dihydro-2H-[1,3]-oxazino[3,2-a]-indol-l0-carboxamid oder ein pharmazeutisch verträgliches Salz davon.

10. Ein Verfahren zur Herstellung von Verbindungen, gemäß Anspruch 1, welches die Reaktion eines entsprechenden Säurederivats mit einem entsprechenden Alkohol oder Amin umfaßt.

11. Ein Arzneimittel, umfassend eine Verbindung, gemäß einem der Ansprüche 1 bis 9 und ein pharmazeutisch verträglicher Träger.

12. Eine Verbindung, gemäß Anspruch 1 zur Verwendung als eine therapeutische Wirksubstanz.

13. Die Verwendung einer Verbindung, gemäß Anspruch 1 zur Herstellung eines Medikaments zur Verwendung als einen 5-HT4-Rezpetorantagonisten.

14. Die Verwendung, gemäß Anspruch 13 zur Verwendung als einen 5-HT₄-Rezeptorantagonisten in der Behandlung oder Prophylaxe von gastroindestinalen Funktionsstörungen, kardiovaskulären Funktionsstörungen und ZNS-Funktionsstörungen.

15. Die Verwendung, gemäß Anspruch 13 zur Verwendung in der Behandlung von Reizkolon, Harninkontinenz oder Vorhofarrhythmien und Schlaganfall.

## Revendications

1. Composé de formule (IA) ou un de ses sels pharmaceutiquement acceptables : 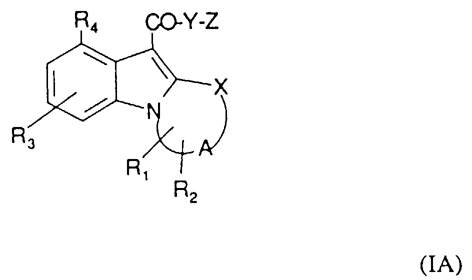 formule dans laquelle
X représente O, S, un groupe SO, SO₂, CH₂, CH ou NR dans lequel R représente l'hydrogène ou un groupe alkyle en C₁ à C₆ ;
A représente une chaîne polyméthylène saturée ou insaturée ayant 2 à 4 atomes de carbone ;
R₁ et R₂ représentent l'hydrogène ou un groupe alkyle en C₁ à C₆ ;
R₃ représente l'hydrogène, un groupe halogéno, amino, nitro ou alkyle en C₁ à C₆ ;
R₄ représente l'hydrogène, un groupe halogéno, alkyle en C₁ à C₆ ou alkoxy en C₁ à C₆ ;
Y représente O ou un groupe NH ;
Z répond à la sous-formule (a) : 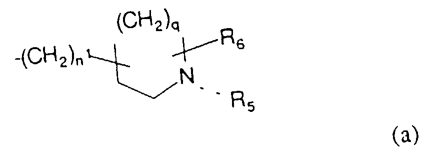 dans laquelle
n¹ est égal à 1, 2, 3 ou 4 ;
q est égal à 0, 1, 2 ou 3 ;
R₅ représente un groupe 3-phénoxypropyle ;
R₆ représente l'hydrogène ou un groupe alkyle en C₁ à C₆.

2. Composé suivant la revendication 1, dans lequel X représente O.

3. Composé suivant la revendication 1 ou 2, dans lequel A représente un groupe -(CH₂)₃-.

4. Composé suivant la revendication 1, 2 ou 3, dans lequel R₁ et R₂ représentent, indépendamment, l'hydrogène ou un groupe méthyle.

5. Composé suivant la revendication 1, 2, 3 ou 4, dans lequel R₃ représente l'hydrogène et R₄ représente l'hydrogène ou un groupe halogéno.

6. Composé suivant l'une quelconque des revendications 1 à 5, dans lequel Y représente un groupe NH.

7. Composé suivant l'une quelconque des revendications 1 à 6, dans lequel Z répond à la sous-formule (a) et (CH₂)ₙ1 est fixé au niveau d'un atome de carbone de l'azacycle.

8. Composé suivant la revendication 7, dans lequel Z représente un groupe 4-pipéridylméthyle N-substitué.

9. N-[(1-(3-phénoxypropyl)-4-pipéridinyl)méthyl]-3,4-dihydro-2H-[1,3]oxazino[3,2-a]indole-10-carboxamide ou un de ses sels pharmaceutiquement acceptables.

10. Procédé pour la préparation de composés suivant la revendication 1, qui comprend la réaction d'un dérivé d'acide approprié avec un alcool ou une amine approprié.

11. Composition pharmaceutique comprenant un composé suivant l'une quelconque des revendications 1 à 9 et un support pharmaceutiquement acceptable.

12. Composé suivant la revendication 1, destiné à être utilisé comme substance thérapeutique active.

13. Utilisation d'un composé suivant la revendication 1, dans la production d'un médicament destiné à être utilisé comme antagoniste des récepteurs 5-HT₄.

14. Utilisation suivant la revendication 13, comme antagoniste des récepteurs 5-HT₄ dans le traitement ou la prophylaxie de troubles gastro-intestinaux, de troubles cardiovasculaires et de troubles du SNC.

15. Utilisation suivant la revendication 13, dans le traitement du syndrome du côlon irritable, de l'incontinence urinaire ou des arythmies atriales et de l'ictus.
